Europäisches Patentamt

European Patent Office

Office européen des brevets

(11) Publication number: **0 068 712**
A1

# EUROPEAN PATENT APPLICATION

(21) Application number: **82303104.2**

(22) Date of filing: **15.06.82**

(51) Int. Cl.³: **A 61 F 5/44**

(30) Priority: **25.06.81 GB 8119585**
**19.01.82 GB 8201402**

(43) Date of publication of application: **05.01.83**
**Bulletin 83/1**

(84) Designated Contracting States: **AT BE CH DE FR GB IT LI LU NL SE**

(71) Applicant: **E.R. Squibb & Sons, Inc., Lawrenceville-Princeton Road, Princeton, N.J. 08540 (US)**

(72) Inventor: **Cochrane, George William, "North Fleet" Chequers Meadow Front Street, South Creake Fakenham Norfolk (GB)**

(74) Representative: **Cook, Anthony John et al, D. YOUNG & CO. 10, Staple Inn, London, WC1V 7RD (GB)**

(54) **Male incontinence device and method of using the same.**

(57) The device comprises an applicator (10), which is a substantially cylindrical device (11), with a peripheral groove (12, 14) at either end, and a condom-like, elastic incontinence device (10) with a hole (18) at its extremity. The incontinence device is rolled up and placed over one end of the applicator with the rolled up portion in the peripheral groove (14) at one end of the applicator (10). Then, the applicator, with the incontinence device thereon is applied over the penis, and a retaining ring (26) which was held by the peripheral groove (12) at the opposite end of the applicator is rolled off the applicator onto the penis immediately to the rear of the corona whereupon the retaining ring insures the retention of the incontinence device on the head of the penis. The applicator is then removed from the penis, and the incontinence device is rolled back over the penis where it serves as a tube to conduct urine away from the wearer.

EP 0 068 712 A1

-1-

## MALE INCONTINENCE DEVICE AND
## METHOD OF USING THE SAME

The present invention relates to a male incontinence device and to a method of using the same.

The prior art is replete with approaches to the problem of incontinence. Many devices have been unsuccessful because they failed to allow the external skin in the genital area to be kept dry. Others failed because of leakage, and yet others failed because the adhesive or sticky tape or binding by which they were affixed caused skin problems for the wearer, e.g, oedema, maceration, soreness, or allergic reactions. Other devices are unsuitable for children where retraction of the penis into the body tissue around the pubic area is likely to occur. Devices which require belts, harnesses, rigid supports, or straps are cumbersome to put on and take off, and, in the case of hospitalized persons, require much use of scarce skilled nursing time and ability.

It is also highly desirable, in the case of an adolescent or grown male, that the occurrence of an erection while wearing the device should not give rise to dislodgement of the device or pain or discomfort to the user, nor should it cause damage to his penis. Many devices are too complex or expensive or difficult to manufacture to be contemplated for widespread use.

From the points of view of simplicity, long term effectiveness, and long term comfort, it is presently believed that a condom-like device of thin flexible material such as latex rubber represents the best way to deal with the many different problems, and an expansible ring of rubber or equivalent stretchable material is a simple, cheap and effective way of retaining the device in position on the penis.

The present invention is an incontinence device comprising a condom having an outlet hole for urine and a stretchable retainer ring arranged to seat immediately behind the corona, the unstretched diameter of the ring being from 55% to 75% of the maximum diameter of the penis upon which the device is to be worn.

Preferably, the unstretched diameter of the ring is from 55% to 65% of the said dimension, and most preferably it is about 60% of the said dimension.

The ring may be separate from, or integral with, the condom.

In one preferred embodiment of the invention, the condom is dimensioned to cover only the head (glans) of the penis. In another preferred embodiment, a length of the condom is folded away from the body over the head of the penis and serves as a conduit for discharged urine.

According to one aspect of the invention, there is provided a method of applying and using a male incontinence devicle in the form of a penis-fitting condom of rubber or equivalent material and having an open end and a generally closed end with a small urine-passage hole therein. The method comprises applying the condom to a penis so that the glans is snugly seated in the generally closed end, applying a stretchable retainer ring to the penis, the strechable retainer ring being external to the condom so that it encircles the penis to the rear of the adjacent the corona, folding back that portion of the condom which terminates in the open end over the ring and over the glans, and connecting the open end to a suitable conduit to take away any excreted urine.

According to another aspect of the invention, there is provided a kit of parts for carrying out such a method. The kit includes a penis-fitting tubular sheath of rubber or equivalent material having an open end and a generally closed end, a retainer ring which is stretchable over the glans of the penis. In its substantially unstretched condition, the retainer ring has a diameter such as to snugly engage behind the corona.

The kit also includes a tubular applicator having an inside diameter (I.D.) greater than the maximum outside diameter (O.D.) of the penis with which it is to be used. The tubular applicator may have a reduced diameter portion at or near one end constructed to have the retainer ring temporarily stretched thereonto.

Further, according to the present invention, there is provided a tubular applicator for applying a condom-like incontinence device to the penis of a user. The applicator comprises a hollow tubular body having an O.D. such that the device can be readily stretched thereover and an I.D. that can accommodate the penis, a first groove at one end thereof upon which a ring located at the mouth of the condom-like device can be stretched, and a second groove at the other end upon which a retainer ring of rubber, or like material, can be stretched.

The invention, in yet a further aspect, consists in a male incontinence device comprising a retainer ring in combination with a tubular elastic sheath dimensioned to be stretched over the penis of the wearer. The sheath has a first portion which fits over the glans and has an orifice therein for the passage of urine. A second portion of length greater than the first portion and integral therewith has an open end surrounded by a stretchable ring.

FIG. 1 shows a retainer ring, an applicator, and a condom-like device according to a first embodiment of the invention in rolled condition ready for application to the applicator;

FIG. 2 shows the applicator bearing the rolled device and the retainer ring;

FIGS. 3A – 3C show stages in the application of the condom-like device to the penis of a user;

FIG. 4 shows a condom-like device worn by a user and connected to a drainage bag;

FIG. 5 shows part of FIG. 4 on a larger scale and illustrates the details of the retainer ring seated immediately behind the corona and the folded-back tubular part of the condom-like device serving as a conduit to conduct urine to a drainage device;

FIG. 6 is a cross-section, on an enlarged scale, taken in an axial plane, through one form of stretchable retainer ring usable in the invention as ring 26 of FIGS. 1 – 5, ring 46 of FIG. 8, ring 64 of FIG. 9, or ring 82 of FIGS. 10 and 11;

FIG. 7 is a view similar to FIG. 6 of another form of stretchable retainer ring;

FIG. 8 diagrammatically illustrates an embodiment of the invention comprising a glans condom used to secure a cap with an outlet tube;

FIG. 9 diagrammatically illustrates a further embodiment of the invention, similar to FIG. 8 but in which the outlet tube on the cap is of flexible, bellows-type construction; and

FIGS. 10 and 11 are two diagrammatic views of a further embodiment of the invention, of simple construction for short term use.

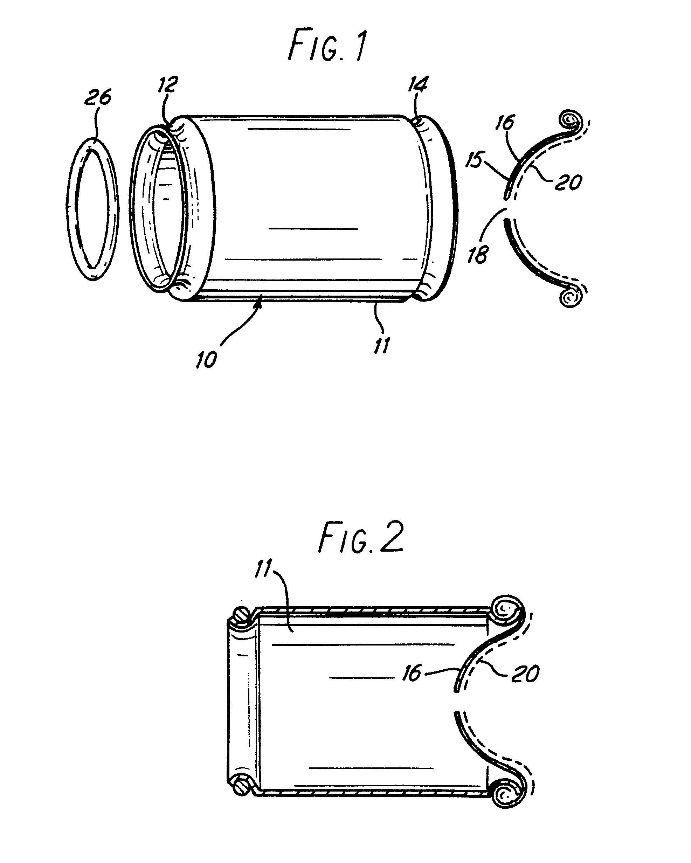

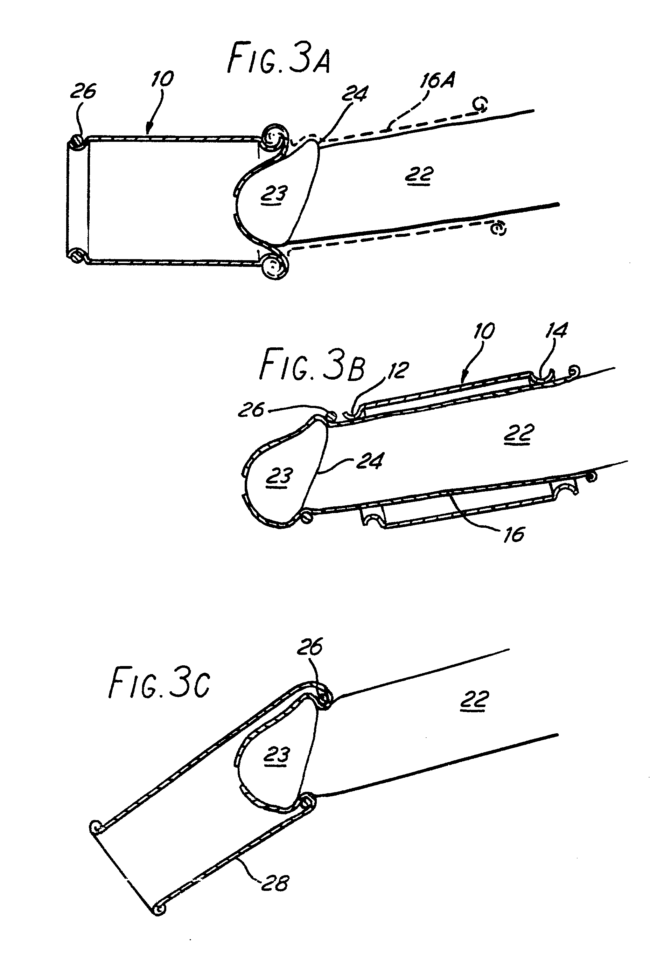

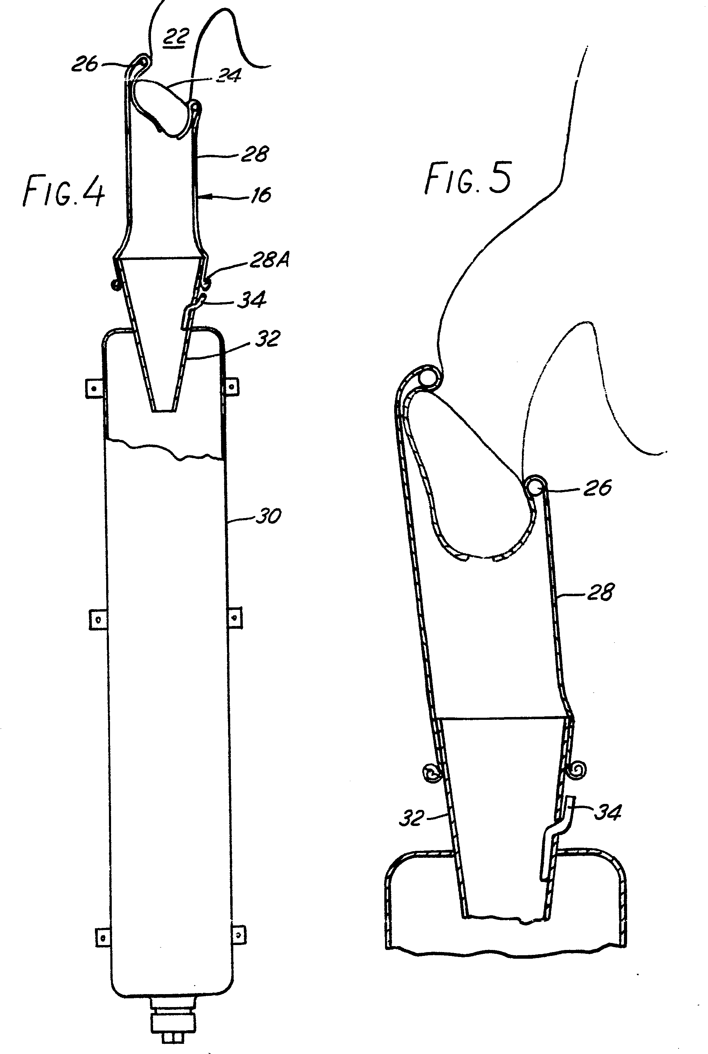

Referring now to FIGS. 1 - 5, an applicator 10 comprises a substantially cylindrical shell 11 having peripheral grooves 12, 14 at opposite ends. A thin-walled, condom-like incontinence device 16, herein called a condom, has a hole 18 in its end and is seen in rolled-up condition in FIGS. 1 - 3. In use, it is stretched gently over the end of the applicator 11 so that its rolled up portion rests in the groove 14, as seen in FIG. 2. As illustrated, the interior of the head region 15 of the condom 16 is coated with an adhesive 20, but this is an optional feature of the invention. It is not an essential feature. Using the applicator 10, as illustrated in FIG. 3, the condom 16 is applied to the head 23 (glans) of the penis 22, and the shank portion of the condom 16 is rolled back along the shaft of the penis 22 as indicated at 16A in FIG. 3. The applicator 10, whose minimum internal diameter is comfortably in excess of the outside diameter of the penis 22 with which it is intended to be used, is then moved up the shaft of the penis 22 until its groove or channel 12 is located just to the rear of the corona 24 of the penis 22, as seen in FIG. 3B. The user can then slip a rubber retaining ring 26 off the applicator 10 onto the penis 22 immediately to the rear of the corona 24, whereupon the ring 26 (which was stretched to be placed on the applicator groove 12) contracts to its unextended diameter and will be snugly and firmly seated on the penis 22 immediately to the rear of the corona 24. The remainder 28 of the sheath of the condom 16 is then pulled "inside out", that is to say pulled away from the wearer's body over the end of the penis 22, to occupy the position illustrated in FIG. 3C. Thus, the tubular part 28 of the sheath serves as a tube to conduct urine away to a suitable container 30, as shown in FIG. 4 or to a connecting tube.

As shown in FIGS. 4 and 5, a connecting cone 32 has the open end of the tubular part 28 stretched over its open end 28A. An air inlet pipe 34 prevents build-up of negative pressure in the system. As an alternative (not shown), the lower end of the cone 32 may be provided with a coupling element of a coupling such as that disclosed and claimed in British Patent Application No. 81 36811, to facilitate connection of the male incontinense device to a drainage bag such as that shown in British Patent Application No. 81 36811.

It has been found in practice that if the ring 26 has an unstretched diameter of appoximately 3/5 the diameter of the penis 22 with which it is to be employed, then it holds the sheath in place securely in the configuration shown in FIG. 5. At the same time it prevents leakage. Yet it is comfortable to wear for long periods of time. The location of the retainer ring 26 immediately behind the corona 24 is, from an anatomical view point and in the inventor's opinion, the ideal location for the ring 26.

This is an important feature of the present invention, and it gives the invention advantages over prior proposals.

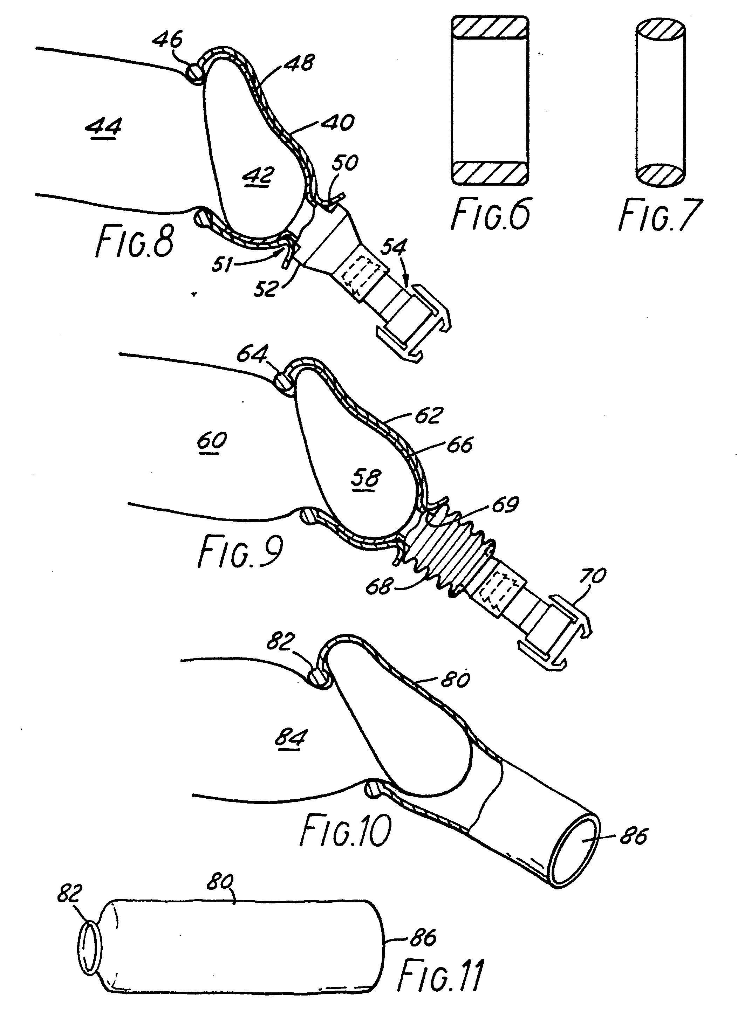

Reference has been made to a relationship of 3/5 or 60Δ for the diameter of the unstretched retainer ring 26 as the fraction, or percentage, respectively, of the maximum diameter of the penis 22 upon which the device is intended to be worn. Naturally, the advantages of the invention can be secured without employing a mathematically exact 60Δ relationship. In many cases it is appropriate and entirely satisfactory if the relationship is from about 55Δ to about 65Δ. The retainer ring 26 may be a true toroid. That is, it may have a circular cross-section taken in a radial plane. However, good results have also been obtained with a ring 26 in the form of a slightly flattened band. That is, a ring 26 having either a rectangular or an oval cross-section, in a radial plane, as illustrated in FIG. 6 and FIG. 7, respectively, can be used.

The normal and average diameter of the adult male penis at the postero-coronal point ("neck") is 30 mm, and the normal and average free-flow diameter of the adult male urethra is 6 mm at the postero-coronal point of the penis.

At this postero-coronal point, the vascular spongy tissue encompassing the urethra (the corpora spongiosum) can be effectively contained with an elastic band placed extragenital, circumferential, and passive to within 3 times the free-flow diameter of the urethra. Under such conditions the occlusive pressure applied to the urethral wall or vascular tissue of the corpora spongiosum would not be sufficient to close down the normal diameter of the urethra.

Therefore 3 times the diameter of the open free flow male urethra is a functionally safe diameter for the unstretched diameter of a stretchable retainer ring 26. This retainer ring 26 will not occlude any substructures, and it will not ride over and off the glans at the corona 24. It provides an excellent fixing means for a glans condom incontinence control device designed to fit sub-prepucially, whether or not the penis 22 has been circumcised. In other words, this type of fixing is suitable for both the circumcised and uncircumcised patient.

As the measuring of urethral diameters by catheter is an invasive technique of acquiring information for calculating retainer ring size, the following practical and non-invasive method of calculation has been devised.

Urethral diameters can be arrived at by measuring the diameter of the penis at the postero-coronal point and using 3/5 of this diameter and 3/4 of this diameter to give practical, useful values for the minimum and maximum retainer ring sizes, repectively, relative to any urethral diameter. The values are given in Table I.

TABLE I

TABLE OF NUMERICAL VALUES FOR
SUITABLE RETAINER RINGS

| | POSTERO-CORONAL DIAMETER | RETAINER RING SIZE | | RELATIVE URETHRAL DIAMETER |
|---|---|---|---|---|
| | | MIN. | MAX. | |
| ADULT MALE | 30mm | 18mm | 22.50mm | 6mm |
| Intermediate sizes if required | | | | |
| ADOLESCENT | 25mm | 15mm | 18.75mm | 5mm |
| Intermediate sizes if required | | | | |
| CHILDREN | ---(20mm | 12mm | 15.00mm | 4mm |
| | (15mm | 9mm | 11.25mm | 3mm |

It is not essential to the invention that the tubular part of the sheath be used to conduct the urine away. A comfortable and effective male incontinence device may have a penis head condom secured by a retainer ring, with the condom in turn serving to hold in position a shaped rubber or plastic conduit member. Such an arrangement is illustrated in FIG. 8, in which a condom 40 covering only the head (glans) 42 of the penis 44 is secured by a retaining ring 46. The retaining ring 46 is shown to be integral with the condom 40, but it could, alternatively, be a separate part. The ring 46, in either case, has the critical diameter relationship, as compared to the penis 44, which has been discussed above. A separate glans cap 48 is held on the penis head 42. The lower part of the glans cap 48 has a peripheral groove 50 and a tubular portion 52. A coupling member 54 may be integral with or secured to the tubular portion 52. The preferred coupling member is either a male or a female coupling element of a connector as disclosed and claimed in our Patent Application No. 81 36811. A tie, tape, a cord, or an elastic band (not shown) surrounds the outside of the condom 40 at the indicated point 51 to improve the security against leakage by holding the condom 40 tightly into the groove 50.

A somewhat similar, but alternative, arrangement is shown in FIG. 9. The head (glans) 58 of a penis 60 is covered by a glans condom 62, held on the glans 58 by a retainer ring 64. The retainer ring 64 is made of rubber other stretchable material, and it may be integral with or secured to the condom 62. A glans cap 66 (which may be made of a medically acceptable and commerically available material known as Plastisol) covers the forward part of the head 58 of the penis 60 and is located within the condom 62. The elasticity of the condom 62 holds the cap 66 snugly and firmly on the glans 58. A conduit member 68 forms part of the cap 64 and is connected to or integral with a coupling element 70.

The conduit member 68 has a peripheral external groove 69. A tie, tape, or an elastic band (not shown) surrounds the condom 62 and holds it in the groove 69 in the same way as described in connection with parts 50 and 51 in FIG. 8. The preferred coupling element is either the male or the female coupling element disclosed and claimed in our Patent Application No. 81 36811. The conduit member 66 is made of a flexible material and in bellows configuration so that it can bend and is longitudinally extensible. Although a short bellows-form tube is illustrated, it may of course be made longer.

A short term emergency male incontinence device is illustrated in FIGS. 10 and 11. A short length of thin-walled tube 80 such as rubber latex is open-ended at both ends and has a resilient ring 82 fixed to or integral with one end. The ring diameter is within the critical range according to the present invention, and the ring is placed immediately behind the corona of a penis 84 in the manner previously described. The open end 86 of the sheath or tube 80 is connected in any convenient way to a bag or another tube. This device is a cheap and simple device which is useful for a short temporary period until a more sophisticated incontinence device (such as that of FIG. 8 or FIG. 9) can be fitted. The ring 82 may be a standard elastic band of appropriate unstretched diameter.

An important advantage of the invention as particularly disclosed herein is that it dispenses completely with supporting straps and harnesses of any kind. This is of particular importance in the case of paraplegics and spina bifida patients with whom all harnesses are likely to cause tissue trauma due to the fact that such patients have not sensation below the waist and are unaware of any pressure being applied or any chafing occurring. Moreover, in the case of wheelchair patients, the proper adjustment of a harness is difficult.

Another advantage of the present invention, is that it covers the smallest area of tissue possible thereby reducing the area where an interface of urine can occur. As is known, the sequel to creation of a urine interface is urine burn and tissue trauma. In bad cases, plastic surgery will be required.

A device for a patient who has no sensation in the pelvic area must also avoid causing pressure and chafing. The consequences if these are not avoided are erosion of tissue, strictures, and tissue trauma fistulas.

For satisfactory results in most practical cases, the following principles should be followed in the selection of and fitting of retainer rings.

I Claim:

1.   An incontinence device for a male comprising:

(a)   a condom-like sheath having a hole formed at its extremity, said condom-like sheath being adapted to be rolled up;

(b)   an applicator comprising a cylindrical tube including means for retaining said condom-like sheath at one end thereof when said condom-like sheath is in its rolled up state; and

(c)   a retaining apparatus adapted to retain said condom-like sheath over the head of a penis when said condom-like sheath is unrolled over a penis.

2.   The incontinence device for a male of Claim 1 wherein said retaining apparatus is selected from the group consisting of an elastic retaining ring, a tie, and a tape.

3.   The incontinence device for a male of Claim 2 wherein said retaining apparatus is an elastic retaining ring.

4.   The incontinence device for a male of Claim 3 wherein said retaining ring has a cross-section, taken in a radial plane, which is selected from the group consisting of a circle, a rectangle, and an oval.

5.     The incontinence device for a male of Claim 3 wherein said retaining ring has an unstretched diameter of about 3 times the diameter of the urethra of the wearer.

6.     The incontinence device for a male of Claim 3 wherein said retaining ring has an unstretched diameter between about 9mm and 22.5mm.

7.     The incontinence device for a male of Claim 3 wherein said retaining ring has an unstretched diameter between about 55Δ and 65Δ of the diameter of the penis of the wearer.

8.     The incontinence device for a male of Claim 1 wherein said means for retaining said retaining apparatus on said applicator comprises a peripheral groove formed around one end of said applicator.

9.    The   method   of   applying   an
incontinence   device   on   a   male   of   the   type
comprising   a   condom-like   sheath   having   a   hole
formed   at   its   extremity,   said   condom-like   sheath
being   adapted   to   be   rolled   up;   an   applicator
comprising   a   cylindrical   tube   including   means   for
retaining   said   condom-like   sheath   at   one   end
thereof   when   said   condom-like   sheath   is   in   its
rolled   up   state;   and   a   retaining   apparatus
adapted   to   retain   said   condom-like   sheath   over
the   head   of   a   penis   when   said   condom-like   sheath
is   unrolled   over   a   penis,   said   method   comprising
the   steps   of:

(a)   rolling   up   said   condom-like   sheath
toward   the   extremity   having   said   hole;

(b)   applying   said   rolled   up   condom-like
sheath   over   one   end   of   said   applicator   and   using
said   means   for   retaining   said   condom-like   sheath
to   retain   it   thereon;

(c)   placing   said   retaining   apparatus
over   the   other   end   of   said   applicator;

(d)   placing   the   applicator,   with   the
incontinence   device   thereon,   over   the   penis   and
unrolling   the   condom-like   sheath   onto   the   penis;

(e)   sliding   said   retaining   apparatus
off   the   applicator   onto   the   penis,   whereupon   the
retaining   ring   holds   the   condom-like   sheath   on
the   head   of   the   penis;

(f)   sliding   the   applicator   off   the
penis;   and

(g)   rolling   the   condom-like   sheath   back
over   the   penis   where   it   serves   as   a   tube   to
conduct   urine   away   from   the   wearer.

10.    The    method    of    applying    an
incontinence device on a male of Claim 9 wherein
said step of sliding said retaining apparatus off
the applicator onto the penis comprises the step
of sliding said retaining ring on the head of the
penis immediately to the rear of the corona.

## FIG.1

26  12  14  16  15  20  18  10  11

## FIG.2

11  16  20

FIG. 3A

FIG. 3B

FIG. 3C

FIG.4

FIG.5

FIG.6

FIG.7

46

48

44

40

42

50

FIG.8

51

52

54

64

62

60

66

58

69

FIG.9

68

70

82

80

84

FIG.10

86

82

80

86

FIG.11

European Patent Office

EUROPEAN SEARCH REPORT

0068712

EP 82303104.2

| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | A 61 F 5/44 |
| X | US - A - 2 789 560 (WEIMER)<br>* Fig. 5,6 *<br>-- | 1,8 | |
| P,A | FR - A1 - 2 482 450 (KEN)<br>& BE-A - 888 768 (28-08-1981)<br>& GB-A -2 075 847 (25-11-1981)<br>& SE-A -81-02808 (14-11-1981)<br>& NL-A -81-02075 (01-12-1981)<br>& DE-A1-3 114 894 (16-06-1982)<br>-- | 1 | |
| A | DE - A - 520 401 (WENDT)<br>-- | | TECHNICAL FIELDS SEARCHED (Int.Cl. 3) |
| A | DE - A - 215 554 (PENIN)<br>-- | | A 61 F 5/00 |
| A | GB - A - 1 304 544 (AB STILLE-WERNER)<br>-- | | |
| A | US - A - 3 742 953 (LEE)<br>---- | | |

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on. or after the filing date
D: document cited in the application
L: document cited for other reasons

&: member of the same patent family, corresponding document

| X | The present search report has been drawn up for all claims | | |
|---|---|---|---|
| Place of search<br>VIENNA | Date of completion of the search<br>06-09-1982 | Examiner<br>EBERLE | |

EPO Form 1503.1   06.78